# EUROPEAN PATENT SPECIFICATION

(11) **EP 3 900 655 B1**
(45) Date of publication and mention of the grant of the patent: **19.03.2025**
(21) Application number: 21168012.9
(22) Date of filing: 13.04.2021
(51) Int. Cl.: A61B 17/70, A61B 5/00, A61B 5/11

(54) **SYSTEM FOR POST-OPERATIVE ASSESSMENT OF SPINAL MOTION AND IMPLANT-BASED STRAIN CORRELATION**
SYSTEM ZUR POSTOPERATIVEN BEWERTUNG DER WIRBELSÄULENBEWEGUNG UND IMPLANTATBASIERTEN BELASTUNGSKORRELATION
SYSTÈME POUR L'ÉVALUATION POST-OPÉRATOIRE DU MOUVEMENT DE LA COLONNE VERTÉBRALE ET LA CORRÉLATION DE LA DÉFORMATION BASÉE SUR L'IMPLANT

(30) Priority: 22.04.2020 US 202016855444
(43) Date of publication of application: 27.10.2021
(73) Proprietor: Warsaw Orthopedic, Inc., Warsaw, IN 46582 (US)
(72) Inventor: METCALF, Newton, Memphis, 38111 (US); FIELDS, Robert A., Memphis, 38117 (US); BENSON, Nicholas, Collierville, 38017 (US); KURIAN, Arjun Siby, Memphis, 38103 (US)
(74) Representative: Viering, Jentschura & Partner mbB Patent- und Rechtsanwälte

(56) References cited:
- US-A1- 2017 079 555
- US-A1- 2020 022 735
- US-A1- 2020 085 366

## Description

### TECHNICAL FIELD

The present disclosure generally relates to a wearable body sensor that is used in connection with one or more spinal implant-based sensors to assess strain data pertaining to spinal implants.

### BACKGROUND

Treatment of spinal disorders, such as degenerative disc disease, disc herniations, scoliosis or other curvature abnormalities, and fractures, often requires surgical treatments. For example, spinal fusion may be used to limit motion between vertebral members. As another example, implants may be used to preserve motion between vertebral members.

A patient's spinal condition is generally evaluated using a combination of patient feedback, imaging technologies and clinician evaluations. Since data pertaining to a patient's movement can contain valuable information about the health of the patient's neurological function and musculoskeletal health, it is desirable to obtain this information in an objective rather than subjective manner. US 2020/022735 A1 discloses a load sensing assembly for a spinal implant which includes a set screw having a central opening that extends from a first end of the set screw toward a second end of the set screw.

### SUMMARY

The invention is defined in the appended claims. In an embodiment, a system for assessing a status of a spinal implant includes a reader device, a wearable body sensor, and a spinal implant. The wearable body sensor includes a first short-range receiver, a first short-range transmitter, and an inertial measurement unit. The wearable body sensor is configured to be positioned over at least a portion of a spine of a wearer, and measure movement information corresponding to spinal motion of the wearer while the wearer performs one or more movements while wearing the wearable body sensor. The spinal implant includes one or more sensors configured to measure implant information comprising one or more characteristics of a fusion status of the spinal implant, a second short-range receiver, and a second short-range transmitter. The wearable body sensor is configured to communicate at least a portion of the movement information to the reader device. The spinal implant is configured to communicate at least a portion of the implant information to the reader device via the second transmitter.

The wearable body sensor may be affixed to the wearer via an adhesive. The wearable body sensor may include a mobile electronic device.

The one or more movements may be part of a protocol.

The one or more sensors may include a load sensing assembly configured to detect a strain experienced by the spinal implant. The one or more sensors may include a pressure sensor. The one or more sensors may include a second inertial measurement unit. The one or more sensors may include a temperature sensor.

The reader device may be configured to transmit at least a portion of the movement information and/or the implant information to one or more electronic devices.

The wearable body sensor may be configured to communicate at least a portion of the movement information to the reader device when the reader device is located within a short-range communication distance from the wearable body sensor. The spinal implant may be configured to communicate at least a portion of the implant information to the reader device when the reader device is located within a short-range communication distance from the spinal implant. The reader device may be further configured to transmit power to the spinal implant.

In an embodiment, a system for assessing a status of a spinal implant includes a reader device, a wearable body sensor in communication with the reader device, and one or more spinal implants. The wearable body sensor includes a first short-range receiver, a first short-range transmitter, and an inertial measurement unit. Each of the one or more spinal implants includes one or more sensors configured to measure implant information comprising one or more characteristics of a fusion status of the spinal implant, a second short-range receiver, and a second short-range transmitter. Each spinal implant is in communication with the reader device.

The wearable body sensor may be configured to be positioned over at least a portion of a spine of a wearer and measure movement information corresponding to spinal motion of the wearer while the wearer performs one or more movements while wearing the wearable body sensor.

The wearable body sensor may be configured to communicate at least a portion of the movement information to the reader device.

Each spinal implant may be configured to communicate at least a portion of the implant information to the reader device via the second transmitter. The wearable body sensor may be affixed to a wearer via an adhesive. The wearable body sensor may be a mobile electronic device.

The one or more movements may be part of a protocol.

The one or more sensors may include a load sensing assembly configured to detect a strain experienced by the spinal implant. The one or more sensors may include a pressure sensor. The one or more sensors may include a second inertial measurement unit. The one or more sensors may include a temperature sensor.

The reader device may be configured to transmit at least a portion of the movement information and/or the implant information to one or more electronic devices. The wearable body sensor may be configured to communicate at least a portion of the movement information to the reader device when the reader device is located within a short-range communication distance from the wearable body sensor.

The spinal implant may be configured to communicate at least a portion of the implant information to the reader device when the reader device is located within a short-range communication distance from the spinal implant.

The reader device may be configured to transmit power to the spinal implant.

### BRIEF DESCRIPTION OF THE DRAWINGS

FIG. 1 illustrates an example spinal evaluation system.
FIGS. 2A and 2B each illustrates an example of a wearable body sensor.
FIG. 3 illustrates a flow chart of an example method of obtaining subject information.
FIG. 4 depicts a block diagram of an example of internal hardware that may be used to contain or implement program instructions according to an embodiment.

### DETAILED DESCRIPTION

In some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior". Generally, similar spatial references of different aspects or components indicate similar spatial orientation and/or positioning, i.e., that each "first end" is situated on or directed towards the same end of the device. Further, the use of various spatial terminology herein should not be interpreted to limit the various insertion techniques or orientations of the implant relative to the positions in the spine.

The following terms shall have, for purposes of this application, the respective meanings set forth below:

A "computing device", "electronic device", or "computer" refers to a device or system that includes a processor and memory. Each device may have its own processor and/or memory, or the processor and/or memory may be shared with other devices as in a virtual machine or container arrangement. The memory will contain or receive programming instructions that, when executed by the processor, cause the electronic device to perform one or more operations according to the programming instructions. Examples of electronic devices include personal computers, servers, mainframes, virtual machines, containers, mobile electronic devices such as smartphones, Internet-connected wearables, tablet computers, laptop computers, and appliances and other devices that can communicate in an Internet-of-things arrangement. In a client-server arrangement, the client device and the server are electronic devices, in which the server contains instructions and/or data that the client device accesses via one or more communications links in one or more communications networks. In a virtual machine arrangement, a server may be an electronic device, and each virtual machine or container also may be considered an electronic device. In the discussion below, a client device, server device, virtual machine or container may be referred to simply as a "device" for brevity. Additional elements that may be included in electronic devices will be discussed below in the context of FIG. 6.

The terms "memory," "computer-readable medium" and "data store" each refer to a non-transitory device on which computer-readable data, programming instructions or both are stored. Unless the context specifically states that a single device is required or that multiple devices are required, the terms "memory," "computer-readable medium" and "data store" include both the singular and plural embodiments, as well as portions of such devices such as memory sectors.

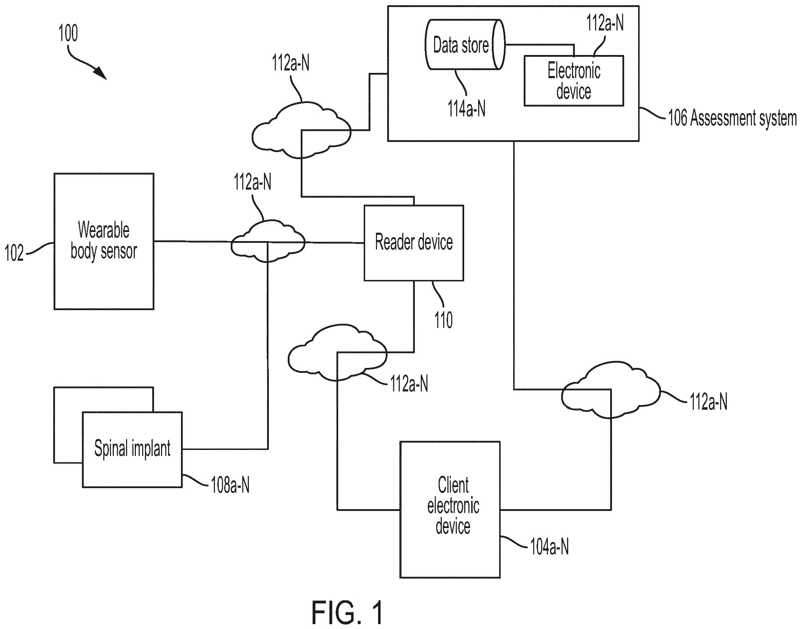

FIG. 1 illustrates an example spinal evaluation system according to an embodiment. As illustrated by FIG. 1, the system **100** includes a wearable body sensor **102,** one or more client electronic devices **104a-N,** an assessment system **106,** one or more spinal implants **108a-N,** and a reader device **110**.

The reader device **110** may be in communication with the wearable body sensor **102** and/or one or more spinal implants **108a-N** via one or more communication networks **112a-N.** The reader device may be in communication with one or more client electronic devices **104a-N** and/or the assessment system **106** via one or more communication networks **112a-N.** In various embodiments, one or more of the client electronic devices **104a-N** may be in communication with the assessment system **106** via one or more communication networks **112a-N.** A communication network **112a-N** may be a local area network (LAN), a wide area network (WAN), a mobile or cellular communication network, an extranet, an intranet, the Internet, a short range communication network and/or the like. Although FIG. 1 shows separate communication networks **112a-N** it is to be understood that these networks, or some combination of these networks, may be implemented as a single communication network.

In various embodiments, the reader device **110** may include a power source, a processing device, and/or one or more communication devices. The power source of the reader device **110** may be a battery. The processing device may be a processor, a microprocessor, and/or the like. The communication devices may include a short-range transmitter, receiver, and/or transceiver. As will be explained in more detail below, a reader device **110** may communicate with the wearable body sensor **102** and/or one or more spinal implants **108a-N** via one or more short range communication protocols. In various embodiments, examples of reader devices **110** may include, without limitation, RFID reader devices, NFC reader devices, and/or the like.

The communication devices may include a transmitter, a receiver, and/or a transceiver that may be used to facilitate wireless communication between the reader device **110** and one or more electronic devices over a wireless network such as, for example, the Internet or an intranet. For example, a reader device may communicate with one or more client electronic devices **104a-N** and/or the assessment system **106**.

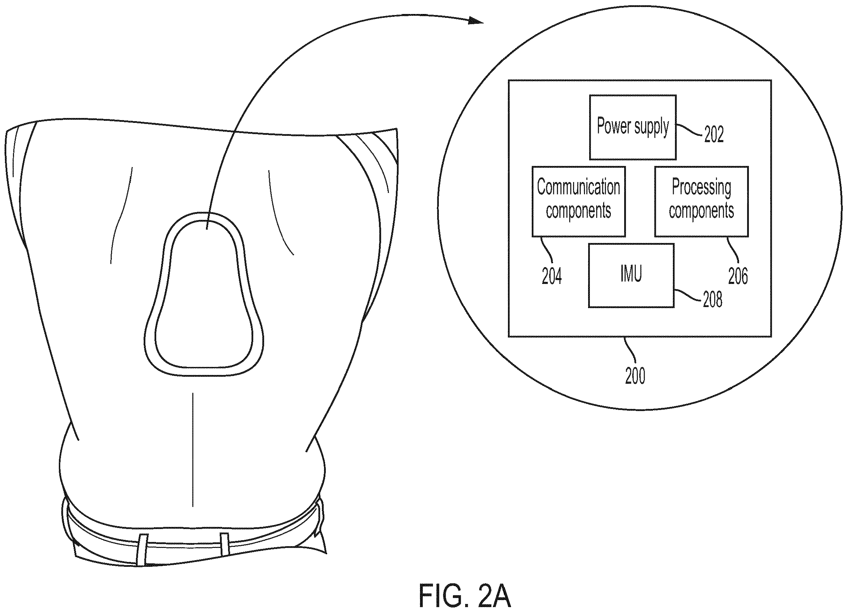

FIG. 2A illustrates an example of a wearable body sensor **102** according to an embodiment. A wearable body sensor **102** may be an electronic device configured to be worn by an individual. For example, as illustrated in FIG. 2A, a wearable body sensor **102** may be a patch. A wearable body sensor **102** may be configured to be worn by an individual across at least a portion of the individual's spine, such as a portion of an individual's lower back, a portion of an individual's middle back, a portion of an individual's upper back, and/or the like. A wearable body sensor **102** may be secured to an individual using an adhesive as illustrated in FIG. 2A. However, it is understood that other ways of securing or affixing a wearable body sensor **102** to an individual may be used within the scope of this disclosure.

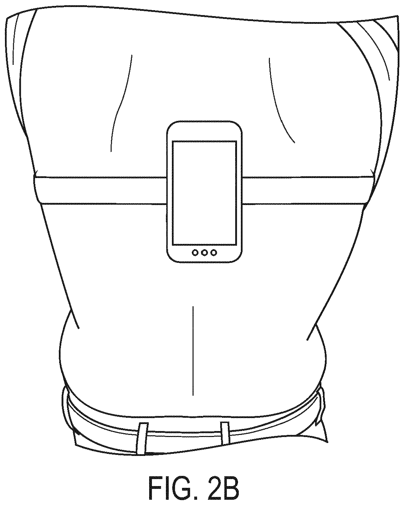

For instance, a wearable body sensor **102** may be secured to an individual via one or more straps, braces, and/or the like. For example, a mobile electronic device, such as a mobile phone, may be a wearable body sensor and may be secured to an individual such that the mobile electronic device is positioned over at least a portion of the individual's spine with one or more straps or belts, as illustrated for example in FIG. 2B. In some embodiments, a wearable body sensor **102** may be part of a wearable garment such as, for example, a harness, a belt, a vest, a shirt, and/or the like. U.S. Patent Application No. 16/132,094. describes example wearable electronic devices and systems which may be used within the scope of this disclosure.

In various embodiments, a wearable body sensor **102** may include an electronics system. As illustrated in FIG. 2, an electronics system **200** may be embedded into at least a portion of the wearable body sensor **102**. The electronics system **200** may include a power supply **202**, one or more communication components **204**, one or more processing components **206**, one or more inertial measurement units (IMU) **208** and/or the like.

The power supply **202** may include a battery. The communication components **204** may include one or more short-range communication components such as, for example, a short-range transmitter, receiver and/or transceiver. The communication components **204** may include one or more other communication components such as, for example, a receiver, a transmitter, and/or a transceiver that may be used to facilitate wireless communication between the electronics system **200** and one or more electronic devices over a wireless network such as, for example, the Internet or an intranet.

The processing components **206** may include one or more processing devices such as, for example, a processor, a microprocessor, and/or the like. The IMUs **208** may include one or more accelerometers, gyroscopes, magnetometers, and/or the like.

The electronics system **200** of a wearable body sensor **102** may be positioned on a portion of the wearable apparatus that overlaps at least a portion of a wearer's spine, or is near to or in proximity to a wearer's spine, when worn. For example, FIG. 2 illustrates an example placement of an electronics system **200** according to an embodiment. However, it is understood that an electronics system, or a portion of an electronics system, may be located elsewhere on the wearable apparatus.

In various embodiments, a wearable body sensor **102** may include one or more integrated circuits, microchips or other memory devices. For instance, a wearable body sensor **102** may include a memory chip that may be removed from the wearable apparatus and inserted into another electronic device in order to transfer data stored on the memory chip. A wearable body sensor **102** may also include firmware and/or a battery, including for example a thin film battery that may be encapsulated or may include a piezo-electronic powering. In various embodiments, a wearable body sensor **102** may include an NFC chip, and RFID chip, and/or the like.

Referring back to FIG. 1, in various embodiments, the wearable body sensor 102 may be in communication with one or more spinal implants **108a-N.** A spinal implant may be a medical device used for the treatment of one or more musculoskeletal disorders. Examples of spinal implants **108a-N** may include, without limitation, vertebral fixation screws, pedicle screws, hooks, cross connectors, offset connectors and related systems for use during various spinal procedures or other orthopedic procedures and that may be used in conjunction with other devices and instruments related to spinal treatment, such as rods, wires, plates, intervertebral implants, and other spinal or orthopedic implants, insertion instruments, specialized instruments such as, for example, delivery devices (including various types of cannula) for the delivery of these various spinal or other implants to the vertebra or other areas within a patient in various directions, and/or a method or methods for treating a spine, such as open procedures, mini-open procedures, or minimally invasive procedures. A spinal implant **108a-N** may include one or more sensors. A sensor may be configured to detect and/or measure one or more characteristics associated with the spinal implant **108a-N.** Example sensors include, without limitation, a load sensing assembly for detecting the strain experienced by an spinal implant. U.S. Patent Application Nos. 16/039,592, 16/395,212, 16/395,216, 16/395,221, and 16/509,285, describe example load sensing assemblies which may be used within the scope of this disclosure. Other examples of sensors may include, without limitation, a pressure sensor, a temperature sensor, an IMU, a gyroscope, and/or the like.

U.S. Patents 6,485,491 and 8,057,519, as well as U.S. Patent Application Nos. 16/039,592, 16/395,212, 16/395,216, 16/395,221, and 16/509,285, describe example spinal implants that may be used within the scope of this disclosure. Other example spinal implants may include, without limitation, interbody fusion devices such as, for example, fusion cages.

A spinal implant **108a-N** may include a receiver, a transmitter, and/or a transceiver. A receiver, a transmitter, and/or a transceiver may be a near-field communication (NFC) or other short-range communication receiver, transmitter, and/or transceiver such as, for example, a radio frequency identification (RFID) coil, an NFC antenna, and/or the like. In various embodiments, a receiver, transmitter, and/or transceiver may be part of an integrated circuit such as, for example, an RFID chip, and NFC chip, and/or the like.

An assessment system **106** includes one or more electronic devices such as, for example, servers and/or one or more data stores. For instance, as shown in FIG. 1, an assessment system **106** may include one or more electronic devices **112a-N** and one or more data stores **114a-N.** A data store **114a-N** may store measured data that is received from one or more sensors such as, for example, movement data, spinal implant performance information, and/or the like. A data store **114a-N** may store movement information that it receives from a wearable apparatus **102**. A data store **114a-N** may store data so that it is correlated to a particular subject.

In various embodiments, one or more sensors of a spinal implant **108a-N** may measure one or more effects of a subject's movement on the spinal implant. This movement or motion may be of a wearer's spinal axis, lower limbs, rotations, bending and/or the like.

A client electronic device **104a-N** may be a smartphone, a tablet, a laptop, a computing device or other electronic device. For instance, a client electronic device **104aN** may be a smartphone or tablet associated with a subject. As another example, a client electronic device **104a-N** may be a smartphone or tablet associated with a clinician, healthcare provider, healthcare entity and/or the like.

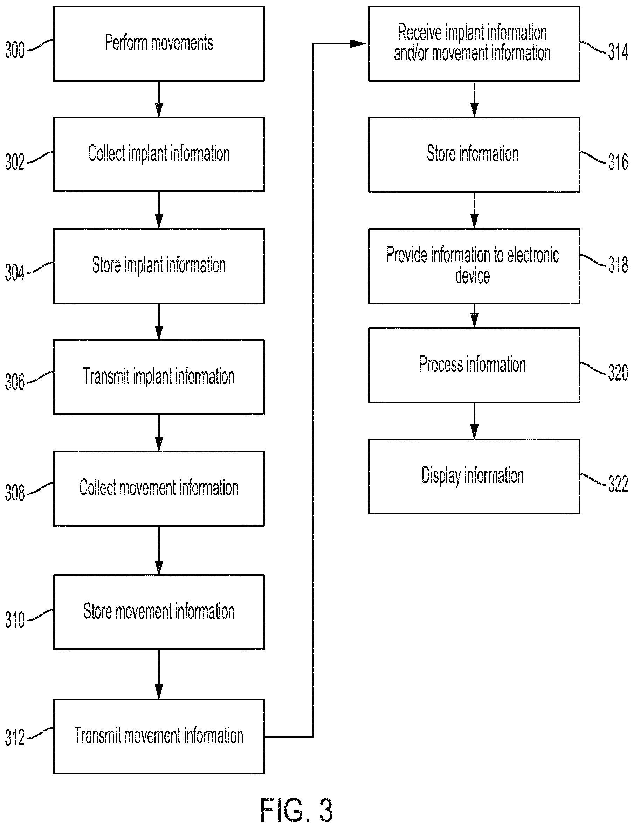

FIG. 3 illustrates a flow chart of an example method of obtaining subject information according to an embodiment. A subject may apply a wearable body sensor. The subject may then perform **300** one or more movements or types of movements while wearing the wearable body sensor.

A subject may wear a wearable body sensor for a limited period of time, such as, for example, in a clinical setting, during an evaluation with a clinician, and/or the like. In this situation, a subject may be asked to perform one or more movements, activities or protocols to gather information about the subject's movement. For instance, a clinician may ask a subject to sit, stand, walk, bend over, rotate, turn, lay down or perform other activities while wearing a wearable body sensor.

While the subject is performing one or more movements, one or more sensors of one or more spinal implants implanted within the subject measure or collect **302** implant information pertaining to one or more characteristics of the associated spinal implant during such movements. For example, a spinal implant may include one or more strain gauges or strain sensors. These sensors may measure strain or one or more strain patterns experienced by the implant during one or more movements or across a range of movements. As another example, a spinal implant may include an IMU which may measure movement information during one or more movements or across a range of movements. In another example, a spinal implant may include a gyroscope which may measure an orientation of a wearer's back or trunk. In another example, a spinal implant may include a pressure sensor that may measure pressure variations on or within the spinal implant. For instance, a set screw of a spinal implant may include a pressure sensor which may measure pressure and/or pressure variations in the chamber of the implant. In another embodiments, a spinal implant may include a temperature sensor which may measure the temperature or temperature variations of the spinal implant or of the area around the spinal implant. Additional and/or alternate sensors and/or measurements may be used and/or made within the scope of this disclosure.

One or more of the spinal implants of the subject may store **304** such implant information. One or more of the spinal implants may transmit **306** at least a portion of such implant information to the reader device when the reader device is within short-range communication distance from the spinal implants. A spinal implant may transmit 306 implant information to a reader device via a short-range transmitter or transceiver.

While the subject is performing one or more movements, the wearable body sensor measures or collects **308** information pertaining to the subject's spinal movement during such movements. Movement information may include one or more characteristics associated with a subject's movement. For example, an IMU may provide information pertaining to the motion of the person wearing the wearable apparatus given the IMU and its placement. The raw x/y/z measurements may, for example, provide only information about the movement of the sensor itself, which may be different from the movement of a wearer. For example, known approaches utilize motion sensors that are integrated into devices such as phones and watches, which move considerably different ways and ways that are independent of their wearers or carriers.

One or more data points of data may have one or more associated parameters such as an associated timestamp, an associated velocity value, an associated barometric pressure value, and associated acceleration value, a rotation value, an orientation value and/or the like.

The wearable body sensor may store **310** such movement information. It may transmit **312** at least a portion of the movement information to the reader device when the reader device is within a short-range communication distance from the body sensor. The body sensor may transmit **312** movement information to a reader device via a short-range transmitter or transceiver.

The reader device may receive **314** implant information from one or more spinal implants and/or movement information from the body sensor. For example, a reader device may receive **314** implant information and/or movement information via a short-range receiver or transceiver. In various embodiments, a reader device may store **316** at least a portion of implant information and/or movement information received from one or more spinal implants and/or the wearable body sensor. A reader device may store **316** at least a portion of received implant information in one or more data stores.

In various embodiments, the wearable body sensor and/or one or more of the spinal implants may send information to the reader device when the reader device is placed within a certain distance from the wearable body sensor and/or one or more of the spinal implants. For example, the wearable body sensor may send information to the reader device when the reader device is located with 3-6 inches from the wearable body sensor. As another example, a spinal implant may send information to the reader device when the reader device is with 3-5 inches from the spinal implant. Other distances and/or distance ranges may be used within the scope of this disclosure. In various embodiments, a wearable body sensor and/or a spinal implant may communicate with a reader device or other electronic device without being interrogated.

The reader device may serve as a remote power source for one or more spinal implants. An electromagnetic field may be generated by the reader device (e.g., a transmitting coil) to transmit power across the skin of a subject to one or more spinal implants. A spinal implant may use the received energy to power or charge the implant.

The reader device is configured to provide **318** at least a portion of the movement information and/or the implant information to an electronic device. For example, a reader device may provide **318** at least a portion of the movement information and/or the implant information to one or more client electronic devices and/or one or more electronic devices associated with an assessment system. In an embodiment, movement information and/or implant information may be provided **318** to an electronic device by removing a memory chip or other data store from a reader device, and connecting it to an electronic device. Alternatively, a reader device may transmit at least a portion of collected implant information and/or movement information to an electronic device via one or more communication networks. In some embodiments, a reader device may transmit information to an electronic device at certain times or intervals. In other embodiments, a reader device may transmit information to an electronic device in response to receiving a request from the electronic device.

One or more electronic devices, such as ones associated with an assessment system, may process **320** at least a portion of the implant information and/or the movement information. In various embodiments, an electronic device may process **320** information to assess one or more characteristics or a status of a spinal implant. Characteristics may be indicative of one or more anomalies or potential issues with one or more spinal implants. In the invention, information is used to determine whether an implant is experiencing unusual or anomalous strain during certain movements. The information is used to detect a fusion status of a subject's spine, whether a spinal implant has failed or malfunctioned, and/or the like. This information may provide valuable insight into the construct and fusion status of an implant.

For example, as a successful spinal implant fusion matures, there is less strain on the hardware of the implant because the bone takes on the load from the implant. If the spinal implant is still experiencing a certain strain level or strain pattern after a certain period of time, it may be a sign of an issue with the fusion status of the implant.

As another example, if a spinal implant is experiencing pressure that exceeds a certain threshold value or is outside of a range of expected values, this may be an indication that there is swelling around the implant. Similarly, if a temperature sensor of a spinal implant measures a temperature that exceeds a certain threshold value or is outside of a range of values, this may be an indication of an infection in an area around the spinal implant.

In various embodiments, an assessment system may cause information pertaining to one or more characteristics or status of a spinal implant to be displayed **322** on one or more electronic devices. For instance, an assessment system may cause an indication of a spinal implant that is experiencing atypical characteristics to be displayed on a tablet associated with the subject's clinician. The displayed information may include one or more measurements from one or more of the spinal implant sensors such as, for example, strain measurements, pressure measurements, temperature measurements, and/or the like. A clinician may use the information to determine whether any changes to any spinal implant need to be made, or to make other treatment recommendations for the wearer.

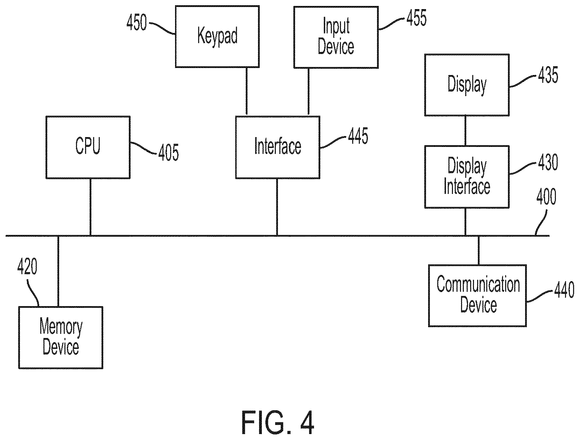

FIG. 4 illustrates example hardware that may be used to contain or implement program instructions. A bus **400** serves as the main information highway interconnecting the other illustrated components of the hardware. CPU **405** is the central processing unit of the system, performing calculations and logic operations required to execute a program. CPU **405**, alone or in conjunction with one or more of the other elements disclosed in FIG. 4, is an example of a processor as such term is used within this disclosure. Read only memory (ROM) and random access memory (RAM) constitute examples of non-transitory computer-readable storage media **420**, memory devices or data stores as such terms are used within this disclosure.

Program instructions, software or interactive modules for providing the interface and performing any querying or analysis associated with one or more data sets may be stored in the memory device **420**. Optionally, the program instructions may be stored on a tangible, non-transitory computer-readable medium such as a compact disk, a digital disk, flash memory, a memory card, a USB drive, an optical disc storage medium and/or other recording medium.

An optional display interface **430** may permit information from the bus **400** to be displayed on the display **435** in audio, visual, graphic or alphanumeric format. Communication with external devices may occur using various communication ports 440. A communication port **440** may be attached to a communications network, such as the Internet or an intranet.

The hardware may also include an interface **445** which allows for receipt of data from input devices such as a keypad **450** or other input device **455** such as a touch screen, a remote control, a pointing device, a video input device and/or an audio input device.

## Claims

1. A system (100) for assessing a status of a spinal implant, the system (100) comprising:
a reader device (110);
a wearable body sensor (102) comprising:
a first short-range receiver,
a first short-range transmitter, and
an inertial measurement unit,
wherein the wearable body sensor (102) is configured to:
be positioned over at least a portion of a spine of a wearer,
measure movement information corresponding to spinal motion of the wearer while the wearer performs one or more movements while wearing the wearable body sensor,
a spinal implant (108) comprising:
one or more sensors configured to measure implant information comprising one or more characteristics of a fusion status of the spinal implant (108) while the wearer performs the one or more movements, wherein the one or more sensors comprise a load sensing assembly configured to detect a strain experienced by the spinal implant (108),
a second short-range receiver, and
a second short-range transmitter, and
an assessment system (106) comprising one or more electronic devices, wherein the assessment system (106) is in communication with the reader device (110),
wherein the wearable body sensor (102) is configured to communicate at least a portion of the movement information to the reader device (110),
wherein the spinal implant (108) is configured to communicate at least a portion of the implant information to the reader device (110) via the second transmitter,
wherein the reader device (110) is configured to provide at least a portion of the movement information and the implant information to the one or more electronic devices associated with the assessment system (106),
wherein the one or more electronic devices are configured to process at least a portion of the implant information and the movement information to assess the one or more characteristics of the fusion status of the spinal implant (108) to thereby allow the determination whether the implant is experiencing unusual or anomalous strain during certain movements.

2. The system (100) of claim 1, wherein the wearable body sensor (102) is configured to be affixed to the wearer via an adhesive.

3. The system (100) of claim 1 or 2, wherein the wearable body sensor (102) comprises a mobile electronic device.

4. The system (100) of any one of claims 1 to 3, wherein the one or more sensors comprises a pressure sensor.

5. The system (100) of any one of claims 1 to 4, wherein the one or more sensors comprises a second inertial measurement unit.

6. The system (100) of any one of claims 1 to 5, wherein the one or more sensors comprises a temperature sensor.

7. The system (100) of any one of claims 1 to 6, wherein the wearable body sensor (102) is configured to communicate at least a portion of the movement information to the reader device (110) when the reader device (110) is located within a short-range communication distance from the wearable body sensor (102).

8. The system (100) of any one of claims 1 to 7, wherein the spinal implant (108) is configured to communicate at least a portion of the implant information to the reader device (110) when the reader device (110) is located within a short-range communication distance from the spinal implant (108).

9. The system (100) of any one of claims 1 to 8, wherein the reader device (110) is further configured to transmit power to the spinal implant (108).

## Patentansprüche

1. System (100) zum Bewerten eines Status eines Wirbelsäulenimplantats, das System (100) umfassend:
eine Lesevorrichtung (110),
einen tragbaren Körpersensor (102), umfassend:
einen ersten Nahbereichsempfänger,
einen ersten Nahbereichssender, und
eine Trägheitsmesseinheit,
wobei der tragbare Körpersensor (102) konfiguriert ist zum:
Positioniertwerden über mindestens einen Teil einer Wirbelsäule eines Trägers,
Messen von Bewegungsinformationen, die einer Wirbelsäulenbewegung des Trägers entsprechen, während der Träger eine oder mehrere Bewegungen durchführt, während er den tragbaren Körpersensor trägt,
ein Wirbelsäulenimplantat (108), umfassend:
einen oder mehrere Sensoren, die konfiguriert sind, um Implantatinformationen zu messen, umfassend eine oder mehrere Eigenschaften eines Fusionsstatus des Wirbelsäulenimplantats (108), während der Träger die eine oder die mehreren Bewegungen durchführt, wobei der eine oder die mehreren Sensoren eine Lasterfassungsanordnung umfassen, die konfiguriert ist, um eine Belastung zu erkennen, die durch das Wirbelsäulenimplantat (108) erfahren wird,
einen zweiten Nahbereichsempfänger, und
einen zweiten Nahbereichssender, und
ein Bewertungssystem (106), umfassend eine oder mehrere elektronische Vorrichtungen, wobei das Bewertungssystem (106) mit der Lesevorrichtung (110) in Verbindung steht,
wobei der tragbare Körpersensor (102) konfiguriert ist, um mindestens einen Teil der Bewegungsinformationen an die Lesevorrichtung (110) zu übermitteln,
wobei das Wirbelsäulenimplantat (108) konfiguriert ist, um mindestens einen Teil der Implantatinformationen über den zweiten Sender an die Lesevorrichtung (110) zu übermitteln,
wobei die Lesevorrichtung (110) konfiguriert ist, um mindestens einen Teil der Bewegungsinformationen und der Implantatinformationen an die eine oder die mehreren elektronischen Vorrichtungen bereitzustellen, die mit dem Bewertungssystem (106) verknüpft sind,
wobei die eine oder die mehreren elektronischen Vorrichtungen konfiguriert sind, um mindestens einen Teil der Implantatinformationen und der Bewegungsinformationen zu verarbeiten, um die eine oder die mehreren Eigenschaften des Fusionsstatus des Wirbelsäulenimplantats (108) zu bewerten, um dadurch die Bestimmung zu ermöglichen, ob das Implantat während bestimmter Bewegungen eine ungewöhnliche oder anomale Belastung erfährt.

2. System (100) nach Anspruch 1, wobei der tragbare Körpersensor (102) konfiguriert ist, um über einen Klebstoff an dem Träger befestigt zu werden.

3. System (100) nach Anspruch 1 oder 2, wobei der tragbare Körpersensor (102) eine mobile elektronische Vorrichtung umfasst.

4. System (100) nach einem der Ansprüche 1 bis 3, wobei der eine oder die mehreren Sensoren einen Drucksensor umfassen.

5. System (100) nach einem der Ansprüche 1 bis 4, wobei der eine oder die mehreren Sensoren eine zweite Trägheitsmesseinheit umfassen.

6. System (100) nach einem der Ansprüche 1 bis 5, wobei der eine oder die mehreren Sensoren einen Temperatursensor umfassen.

7. System (100) nach einem der Ansprüche 1 bis 6, wobei der tragbare Körpersensor (102) konfiguriert ist, um mindestens einen Teil der Bewegungsinformationen an die Lesevorrichtung (110) zu übermitteln, wenn sich die Lesevorrichtung (110) innerhalb einer Nahbereichskommunikationsdistanz von dem tragbaren Körpersensor (102) befindet.

8. System (100) nach einem der Ansprüche 1 bis 7, wobei das Wirbelsäulenimplantat (108) konfiguriert ist, um mindestens einen Teil der Implantatinformationen an die Lesevorrichtung (110) zu übermitteln, wenn sich die Lesevorrichtung (110) innerhalb einer Nahbereichskommunikationsdistanz von dem Wirbelsäulenimplantat (108) befindet.

9. System (100) nach einem der Ansprüche 1 bis 8, wobei die Lesevorrichtung (110) ferner konfiguriert ist, um Energie an das Wirbelsäulenimplantat (108) zu übertragen.

## Revendications

1. Système (100) permettant d'évaluer un état d'un implant rachidien, le système (100) comprenant :
un dispositif d'affichage (110) ;
un capteur corporel portable (102) comprenant :
un premier récepteur à courte portée,
un premier émetteur à courte portée, et
une unité de mesure inertielle,
dans lequel le capteur corporel portable (102) est configuré pour :
être positionné sur au moins une partie d'une colonne vertébrale d'un porteur,
mesurer les informations de mouvement correspondant au mouvement de la colonne vertébrale du porteur lorsque le porteur effectue un ou plusieurs mouvements alors qu'il porte le capteur corporel portable,
un implant rachidien (108) comprenant :
un ou plusieurs capteurs configurés pour mesurer les informations relatives à l'implant comprenant une ou plusieurs caractéristiques d'un état de fusion de l'implant rachidien (108) pendant que le porteur effectue le ou les mouvements, dans lesquels le ou les capteurs comprennent un ensemble de détection de charge configuré pour détecter une contrainte subie par l'implant rachidien (108),
un second récepteur à courte portée, et
un second émetteur à courte portée, et
un système d'évaluation (106) comprenant un ou plusieurs dispositifs électroniques, dans lequel le système d'évaluation (106) est en communication avec le dispositif de lecture (110),
dans lequel le capteur corporel portable (102) est configuré pour communiquer au moins une partie des informations de mouvement au dispositif de lecture (110),
dans lequel l'implant rachidien (108) est configuré pour communiquer au moins une partie des informations relatives à l'implant au dispositif de lecture (110) par l'intermédiaire du second émetteur,
dans lequel le dispositif de lecture (110) est configuré pour fournir au moins une partie des informations relatives au mouvement et à l'implant au ou aux dispositifs électroniques associés au système d'évaluation (106),
dans lequel le ou les dispositifs électroniques sont configurés pour traiter au moins une partie des informations relatives à l'implant et au mouvement afin d'évaluer la ou les caractéristiques de l'état de fusion de l'implant rachidien (108) et de permettre de ce fait de déterminer si l'implant subit une contrainte inhabituelle ou anormale au cours de certains mouvements.

2. Système (100) selon la revendication 1, dans lequel le capteur corporel portable (102) est configuré pour être fixé au porteur par l'intermédiaire d'un adhésif.

3. Système (100) selon la revendication 1 ou 2, dans lequel le capteur corporel portable (102) comprend un dispositif électronique mobile.

4. Système (100) selon l'une quelconque des revendications 1 à 3, dans lequel le ou les capteurs comprennent un capteur de pression.

5. Système (100) selon l'une quelconque des revendications 1 à 4, dans lequel le ou les capteurs comprennent une seconde unité de mesure inertielle.

6. Système (100) selon l'une quelconque des revendications 1 à 5, dans lequel le ou les capteurs comprennent un capteur de température.

7. Système (100) selon l'une quelconque des revendications 1 à 6, dans lequel le capteur corporel portable (102) est configuré pour communiquer au moins une partie des informations de mouvement au dispositif de lecture (110) lorsque le dispositif de lecture (110) est situé à une distance de communication à courte portée du capteur corporel portable (102).

8. Système (100) selon l'une quelconque des revendications 1 à 7, dans lequel l'implant rachidien (108) est configuré pour communiquer au moins une partie des informations relatives à l'implant au dispositif de lecture (110) lorsque le dispositif de lecture (110) est situé à une distance de communication à courte portée de l'implant rachidien (108).

9. Système (100) selon l'une quelconque des revendications 1 à 8, dans lequel le dispositif de lecture (110) est en outre configuré pour transmettre de l'énergie à l'implant rachidien (108).
